# EUROPÄISCHE PATENTANMELDUNG

(11) **EP 1 219 286 A1**
(43) Veröffentlichungstag der Anmeldung: **03.07.2002**
(21) Anmeldenummer: 01130347.6
(22) Anmeldetag: 19.12.2001
(51) Int. Cl.: A61K 7/26

(54) **Verwendung von Bestandteilen der Kakaofrucht zur Herstellung von Backwaren und/oder Teigwaren zur Hemmung von Karies**

(30) Priorität: 19.12.2000 DE 10063235
(71) Anmelder: Höss, Andreas, 86470 Thannhausen (DE)
(72) Erfinder: Höss, Andreas, 86470 Thannhausen (DE)
(74) Vertreter: Winter, Brandl, Fürniss, Hübner, Röss, Kaiser, Polte Partnerschaft

(57) **Zusammenfassung**

Die vorliegende Erfindung betrifft die Verwendung von Bestandteilen der Kakaofrucht zur Herstellung von Backwaren und/oder Teigwaren zur Hemmung von Karies.

Beschrieben werden u.a. Backwaren und Teigwaren, die dadurch gekennzeichnet sind, daß zumindest ein Teil der verwendeten Rohstoffe durch reine Kakaoschale und/oder Kakaoschalenextrakt ersetzt ist. Kakaoschale ist für ihre karieshemmende Wirkung bekannt, so daß eine hiermit versetzte Backware bzw. Teigware Karies vorbeugen kann.

## Beschreibung

Die vorliegende Erfindung betrifft die Verwendung von Bestandteilen der Kakaofrucht zur Herstellung von Backwaren und/oder Teigwaren zur Hemmung von Karies.

Es ist bekannt, daß bestimmte Pflanzen bzw. ihre Inhaltsstoffe *Streptococcus mutans* und dessen Fähigkeit, das klebende Molekül Glukan zu produzieren, stark hemmen. Dieses Molekül ermöglicht es diesem sowie anderen säurebildenden Mikroorganismen als Plaque an den Zähnen zu haften und dort durch ihre Milchsäurebildung Karies zu verursachen. Aufgrund dieser Eigenschaft ist *Streptococcus mutans* unter den etwa 500 Bakterienstämmen im menschlichen Mund derjenige, der für die meisten Schäden an den Zähnen verantwortlich ist. Besonders wirksam gegen den oben beschriebenen Mechanismus haben sich die Bestandteile bzw. die Inhaltsstoffe der Kakaofrucht, insbesondere der Kakaoschale erwiesen.

Die Kakaoschale, d.h. die Schale der Kakaobohne, wird bei der Herstellung z.B. von Schokolade im großen Maßstab als "Abfallprodukt" erhalten und verworfen. Somit stellt gerade die Kakaoschale einen billigen und von daher besonders vorteilhaften Bestandteil der Kakaofrucht dar.

Es wurde bereits vorgeschlagen, Kakaoschale bzw. Kakaoschalenextrakt in Zahnpasten und Mundspülungen zur Kariesprophylaxe anzuwenden.

Die Darreichung in Form von Zahnpasta bringt jedoch erhebliche Nachteile mit sich. So müßten für eine optimale Mundhygiene nach jeder Mahlzeit und nach jedem Verzehr vergärbarer Zucker die Zähne mit Zahnpasta geputzt und die Zahnzwischenräume mit Dentalseide gereinigt werden. Dies erfordert eine große Disziplin und einen sowohl zeitlich als auch finanziell nicht unerheblichen Aufwand. Eine optimale Zahnpflege ist im Alltag kaum praktikabel.

Angesichts dieser Tatsache liegt der vorliegenden Erfindung die Aufgabe zugrunde, diese Wirkstoffe für die Kariesprophylaxe der Mundflora auf eine bequemere Art und Weise und vor allem in zeitlicher und örtlicher Übereinstimmung mit der Nahrungsaufnahme zuzuführen, um schon die beginnende Plaquebildung und die damit einhergehende Säureproduktion effektiv zu hemmen.

Diese Aufgabe wird mit dem Gegenstand des unabhängigen Anspruchs gelöst.

Gegenstand der vorliegenden Erfindung ist die Verwendung von Bestandteilen der Kakaofrucht zur Herstellung von Backwaren und/oder Teigwaren zur Hemmung von Karies.

Die abhängigen Ansprüche geben bevorzugte Ausführungsformen der vorliegenden Erfindung an.

Vorzugsweise werden die Bestandteile der Kakaofrucht aus natürlichem Kakaopulver, entöltem Kakaopulver, alkalisiertem Kakaopulver, Kakaobohnen, Kakaobohnenstücken, Kakaoschalen, Kakaoschalenstücken Kakaoschalenraspeln, Kakaoschalenpulver, oder Mischungen und/oder Extrakten davon ausgewählt, insbesondere aus Kakaoschalen, Kakaoschalenstücken, Kakaoschalenraspeln, Kakaoschalenpulver oder Mischungen und/oder Extrakten davon.

Die Extrakte werden vorzugsweise aus wäßrigen Extrakten, alkoholischen Extrakten, alkoholisch-wäßrigen Extrakten und Extrakten mit organischen Lösungsmitteln ausgewählt.

Es hat sich als besonders vorteilhaft erwiesen, daß zumindest ein Teil der zur Herstellung der Backwaren und/oder Teigwaren verwendeten Rohstoffe, insbesondere des Mehls und/oder der Backmittel, durch reine Kakaoschale und/oder Kakaoschalenextrakt ersetzt ist.

Gemäß der vorliegenden Erfindung wird in einer Teigzubereitung für eine Backware bzw. Teigware vorzugsweise zumindest eine Teilmenge des Mehls und/oder des Backmittels durch reine Kakaoschale und/oder Kakaoschalenextrakt substituiert.

Die karieshemmenden Wirkstoffe sind gegenüber den Milieubedingungen im Teig, das heißt den Enzymen, den reduzierenden und oxidierenden Substanzen und dem mikrobiellen Abbau, chemisch stabil. Im Temperaturbereich von ca. -40 °C (Frosterlagerung der Teiglinge) bis ca. 98° C (Kerntemperatur beim Backen) konnte keine negative Beeinflussung hinsichtlich der Wirksamkeit festgestellt werden.

Sowohl hinsichtlich der Be- und Verarbeitbarkeit als auch der Backfähigkeit der mit Bestandteilen der Kakaofrucht, insbesondere der Kakaoschale, hergestellten Teigzubereitungen zeigten sich keine negativen Auswirkungen.

Zur Verwirklichung der erfindungsgemäßen Lehre genügt es, die karieshemmenden Wirkstoffe der Kakaofrucht als Reinsubstanzen, in Lösung, als Extrakt oder auf geeignete Trägersubstanzen wie z.B. Stärkepuder aufgebracht zu der Teigzubereitung zuzusetzen.

Im Falle von Kakaoschale erfolgt die Zugabe in vorteilhafter Weise dadurch, daß die Kakaoschale anstelle eines Anteils des Mehls und/oder des verwendeten Backmittels zugesetzt wird.

Neben der Einbringung der Wirkstoffe bringt diese Substitution eines Teiles des Mehls/des Backmittels durch Kakaoschale weitere Vorteile mit sich:

So ergibt sich durch die Verwendung von Kakaoschale eine interessante Färbung der Gebäcke, eine gute Geschmacksabrundung sowie eine angenehme Frische im Aroma. Geraspelte Kakaoschale bedingt ein interessantes, nicht zu sandiges "Mouthfeeling".

Weiterhin zeigten sich Einflüsse auf die teigrheologischen Eigenschaften. Die Teige wiesen eine gute Stabilität, eine etwas trockene Beschaffenheit sowie eine geringe Teigerweichung auf, was für eine gute Be- und Verarbeitbarkeit und eine große Gärtoleranz sorgt. Dadurch kann auf teigstabilisierende Zusatzstoffe, insbesondere Ascorbinsäure weitgehend verzichtet werden.

Die oben genannten Bestandteile der Kakaofrucht, insbesondere der Kakaoschale stellen somit ideale Zusatzstoffe für Backwaren und/oder Teigwaren mit karieshemmender Wirkung dar.

Es versteht sich, daß die Lehre der vorliegenden Erfindung auf eine Vielzahl unterschiedlicher Backwaren, Dauerbackwaren und Teigwaren anwendbar ist, also zum Beispiel Brot aus verschiedenen Getreidesorten, Kleingebäck wie Brötchen, Brezeln und dergleichen, Stangenweißbrot und anderes Formgebäck, süße Backwaren wie Plunder- und Blätterteigteilchen, Rührkuchen und dergleichen. Angesichts der karieshemmenden Wirkung der Kakaoschale liegt ein sehr großer Vorteil der Erfindung im Einsatz sowohl im Snack- und Knabberartikelbereich als auch bei Teigwaren wie beispielsweise Nudeln, da besonders beim Außerhausverzehr und bei Zwischenmahlzeiten eine praktikable Mundhygiene nicht möglich ist.

Nachstehend soll die Erfindung anhand von konkreten Beispielen näher erläutert werden, wobei diese Beispiele keinen einschränkenden, sondern lediglich einen illustrierenden Charakter aufweisen.

### Beispiel 1

Teigzubereitung für ein Mischbrot (50/50 Weizen/Roggen)

Die Mischung der Rohstoffe enthält 500 g Weizenmehl Type 1050, 500 g Roggenmehl Type 1150, 20 g Kochsalz, 10 g Backmittel Doppelfrisch®,5 g Backmittel P1®, 40 g Backhefe und 50 g geraspelte bzw. pulverisierte Kakaoschale und ca. 700 ml Wasser. Der Teig wird auf übliche Weise geknetet, nach 30 min Teigruhe in 870 g schwere Stücke geteilt, gewirkt, in Kipfform aufgearbeitet und nach 45 min Stückgare bei 35° C und 80% rel. Luftfeuchte bei 240° C nach Schwadengabe bei geöffnetem Zug gebacken. Als Ergebnis konnte hinsichtlich Volumen, Form, Porung und Krumenbeschaffenheit ein typisches Mischbrot erhalten werden. Die Krume wies eine angenehme Farbe auf. Das Aroma des Brotes war nicht untypisch, hatte jedoch eine interessante, frische, adstringierende Komponente.

Durch weitere umfangreiche Untersuchungen konnte gezeigt werden, daß unter Einhaltung bestimmter Eckparameter durch entsprechende enzymatische Einstellung des Mehls und Anpassung der Zugabe von Genußsäuren wie z.B. Milchsäure und rheologiebeeinflussenden Zusatzstoffen wie z.B. Sojaprotein in der Praxis keine Einschränkungen zu erwarten sind. Die Mengen der Zusatzstoffe konnte meist verringert werden, was als weiteren positiven Effekt ein naturbelasseneres Produkt zur Folge hat. Bei der Herstellung von Sauerteigbroten zeigte sich überraschenderweise ein nivellierender Effekt bezüglich der optimalen Sauerteigmenge: Zu Vergleichsbroten ohne Kakaoschale konnte sowohl mit verringerter als auch erhöhter Sauerteigmenge ein gutes Backergebnis erzielt werden.

Der hervortretende bittere Geschmack, insbesondere bei stark erhöhter Kakaoschalenzugabe ließ sich abmildern durch die Zugabe von Molke und/oder Milch in auf die typischen Gebäckeigenschaften abgestimmter Menge.

Getrocknetes Kakaoschalenpulver ließ sich besonders vorteilhaft verarbeiten, wenn es in Speiseöl, beispielsweise Olivenöl, einige Stunden lang eingeweicht wurde. Dies verringerte die Sandigkeit der Backwaren und beeinflußte auch das Aroma positiv.

Für die Dekoration der Gebäckoberfläche, z.B. bei Gewürzbroten stellte sich die Verwendung von grob gemahlenen Kakaoschalen oder im Gemenge mit geschrotetem Getreide als gut praktikabel dar.

Wie aus den Ausführungen hervorgeht, ist die Zugabe von Kakaoschale und/oder Kakaoschalenextrakt nicht auf das oben aufgeführte Beispiel beschränkt: Der Gedanke der Erfindung kann gleichermaßen auch bei anderen Backwaren und Backmischungen Einsatz finden. Beispielhaft seien hier Gebäcke mit/aus unterschiedlichen Getreidearten wie Hartweizen, Dinkel, Emmer, Einkorn, Mais, Reis, Hirse und Hafer aber auch Buchweizen, und mit weiteren Zuschlägen wie Ölsaaten, Gewürzen und Kräutern, Früchten und Fruchtzubereitungen, Nüssen, Hülsenfrüchte, Gemüse oder alkaloidhaltigen Genußmitteln genannt.

Darüber hinaus liegt es im Rahmen der vorliegenden Erfindung, Backwaren und/oder Teigwaren sowie fertige Backmischungen herzustellen, denen Kakaoschale beziehungsweise ein Extrakt in gemahlener, geriebener, geraspelter, flüssiger, pastöser, pulveriger agglomerierter oder granulierter Form zugegeben und/oder beigefügt ist. Insbesondere bei erwünschter langer Haltbarkeit ist eine separate Verpackung feuchter Reibsel oder flüssigen Extraktes denkbar.

### Beispiel 2

Weizenkleingebäck (Brötchen etc.)

Aus 1000 g Weizenmehl Type 550, 30 g Enzymalt®, 20 g Kochsalz, 50 g Hefe, 100 g Kakaoextrakt (wäßrig, dickflüssig/ pastös) und ca. 550 g Wasser wurden zu einem üblichen Brötchenteig geknetet. Nach 10 Minuten Teigruhe und 15 Minuten Ballengare wurde der Teig in 30 Teile geteilt und geschliffen. Nach einer Gärzeit von 50 Minuten wurden diese bei Vollgare unter Schwadengabe bei 240° C normal ausgebacken. Die so hergestellten Rundstücke wiesen ein normales Gebäckvolumen, eine übliche Rösche und eine etwas straffe Krume auf.

### Beispiel 3

### Plundergebäck

Aus 1000 Weizenmehl Type 550, 80 g Kakaoschalenpulver, 100 g Backmargarine, 100 g Zucker, 200 g Vollei, 15 g Salz, 45 g Hefe und ca. 300 g Vollmilch wurde ein nicht zu intensiv gekneteter Teig (Spiralkneter 1. Gang, 8 Minuten) bereitet. Dieser wurde nach 10 Min. Entspannung dreimal "einfach" mit 600 g Ziehfett touriert. Nach einer weiteren Teigruhe wurde der Teig auf 10 mm ausgerollt, zu Teigdreiecken (11 cm, 18 cm) geschnitten, mit Schokofüllung gefüllt und gewickelt. Die Hörnchenteigling wurden dann bei Vollgare bei 220° C gebacken, Nach dem Backen wurden diese mit Aprikosengelee abgeglänzt und mit Kakaoschalenraspeln bestreut.

### Beispiel 4

### Kartoffelbrot

Aus. 1000 Weizenmehl Type 1050, 100 g Kakaoschalenpüree, 250 g Nasskloßteig "Halb-und-Halb"®, 20 g Weißbrotbackmittel, 20 g Salz, 30 g Hefe und ca. 500 g Wasser wurde ein intensiv gekneteter Teig bereitet. Dieser wurde nach 20 Min. Teigruhe zu 600 g Portionen abgewogen, gewirkt, in Kipfform aufgearbeitet und bei 250 °C fallend auf 200 °C mit anfänglicher Schwadengabe ca. 40 Min. ausgebacken.

Bei dem so hergestellten Kartoffelbrotteig fiel angenehm auf, daß das im Vergleich zu kartoffelhaltigen Teigen übliche Nachlassen nur sehr schwach ausgeprägt war, und auch die Teigoberfläche nicht zum bekannten Schmieren neigte.

### Beispiel 5

### Teigwaren

Aus 1000 g Hartweizengries, 100 g Kakaoschalenpulver, 10 g Kochsalz und 350 ml Wasser wurde ein Nudelteig hergestellt. Dieser wurde nach 1 Stunde Quellzeit zu 1 mm dünnen Bahnen ausgerollt und anschließend zu Bandnudeln in Schneidewalzen geschnitten. Durch übliches 12minütiges "Köcheln" in Salzwasser konnten bissfeste Nudeln erhalten werden. Durch Kochen der Nudeln in einem Sud aus Wasser und geraspelten Kakaoschalen in einem Kochnetz (50 g je Liter Wasser) konnte die karieshemmende Wirkung nochmals weiter gesteigert werden.

Weiterhin ist es im Rahmen der vorliegenden Erfindung möglich, Convenienceprodukte auf Grundlage der erfindungsgemäßen Lehre herzustellen. Derartige Produkte sind lagerfähige Fertigteige, z.B. für Pizzen oder Croissants und vor-, teil- oder halbgebackene Teiglinge wie beispielsweise "Sonntagsbrötchen". Diese Produkte erreichen beim Vorbacken nur Kerntemperaturen, die in Verbindung mit der kurzen Heißhaltezeit keine sichere Pasteurisation darstellen. Dadurch werden die Backwaren besonders im Kern mikrobiologisch nicht oder nur ungenügend haltbar gemacht, so daß der Einsatz von Konservierungsstoffen notwendig ist, um die Lagerfähigkeit sicherzustellen. Durch die mikroorganismenhemmende Wirkung von Kakaoschale und/oder Kakaoschalenextrakt ergibt sich ein synergistischer Effekt auf andere konservierende Zusatzstoffe, so daß deren Gehalt verringert werden kann. Bei Schnittbrot ist aufgrund der stark vergrößerten Oberfläche das Risiko mikrobieller Kontamination besonders groß, so daß nur durch entsprechend hohe Konservierungsstoff- zugaben und/oder Pasteurisation oder Sterilisation die Produktsicherheit gewährleistet werden kann. Durch die Wirkstoffe der Kakaoschale läßt sich entsprechend dem o. g. Synergismus die Zugabe von Konservierungsstoffen verringern bzw. die thermische Belastung des Produktes bei der Hitzekonservierung abmildern. Somit kann ein schonender hergestelltes Schnittbrot erhalten werden.

## Patentansprüche

1. Verwendung von Bestandteilen der Kakaofrucht zur Herstellung von Backwaren und/oder Teigwaren zur Hemmung von Karies.

2. Verwendung gemäß Anspruch 1, **dadurch gekennzeichnet, daß** die Bestandteile der Kakaofrucht aus natürlichem Kakaopulver, entöltem Kakaopulver, alkalisiertem Kakaopulver, Kakaobohnen, Kakaobohnenstücken, Kakaoschalen, Kakaoschalenstücken, Kakaoschalenraspeln, Kakaoschalenpulver, oder Mischungen und/oder Extrakten davon ausgewählt werden.

3. Verwendung gemäß einem der Ansprüche 1 oder 2, **dadurch gekennzeichnet, daß** die Bestandteile der Kakaofrucht aus Kakaoschalen, Kakaoschalenstücken, Kakaoschalenraspeln, Kakaoschalenpulver oder Mischungen und/oder Extrakten davon ausgewählt werden.

4. Verwendung gemäß einem der Ansprüche 2 oder 3, **dadurch gekennzeichnet, daß** die Extrakte aus wäßrigen Extrakten, alkoholischen Extrakten, alkoholisch-wäßrigen Extrakten und Extrakten mit organischen Lösungsmitteln ausgewählt werden.

5. Verwendung gemäß einem der Ansprüche 1 bis 4, **dadurch gekennzeichnet, daß** zumindest ein Teil der zur Herstellung der Backwaren und/oder Teigwaren verwendeten Rohstoffe, insbesondere des Mehls und/oder der Backmittel, durch reine Kakaoschale und/oder Kakaoschalenextrakt ersetzt ist.
